# EUROPEAN PATENT APPLICATION

(11) **EP 3 184 508 A1**
(43) Date of publication of application: **28.06.2017**
(21) Application number: 15202134.1
(22) Date of filing: 22.12.2015
(51) Int. Cl.: C07C 409/20, C07C 409/22, C07C 409/40

(54) **LOW TEMPERATURE RADICAL INITIATOR SYSTEM AND PROCESSES MAKING USE THEREOF**

(71) Applicant: Studiengesellschaft Kohle MbH, 45470 Mülheim an der Ruhr (DE); Universiteit Hasselt, 3500 Hasselt (BE)
(72) Inventor: Klussmann, Martin, 40125 Duesseldorf (DE); Schweitzer-Chaput, Bertrand, 43005 Tarragona (ES); Vandenbergh, Joke, 3700 Tongeren (BE); Junkers, Thomas, 3590 Diepenbeek (BE)

(57) **Abstract**

The present invention refers to a process making use of a radical initiator system comprising peroxy compounds, in particular peroxyketals, in the presence of an acid catalyst to initiate radical processes at low temperature, such as room temperature and below.

## Description

The present invention refers to a process making use of an initiator system comprising certain peroxide compounds, in particular peroxyketals, in the presence of an acid catalyst to initiate radical processes at low temperature, and the initiator system.

Radical chemistry classically relies on the use of initiators to generate free radical species by the thermal homolytic scission of weak chemical bonds. The generated free radical species can then initiate a variety of radical reactions that are of general interest to the chemical community, and of particular relevance to the polymer industry, but also find an increasingly large number of applications in organic synthesis. In the rest of this document, an initiator system, or initiator, is to be understood as a chemical compound or combination of chemical compounds capable of generating initiating radical species under relevant experimental conditions. An overview of classical initiator systems can be found in "Lalevée, J. and Fouassier, J. P. 2012. Overview of Radical Initiation. Encyclopedia of Radicals in Chemistry, Biology and Materials".

The most represented class of initiators are peroxide compounds, wherein a weak O-O bond is broken to generate free radicals, and azobis compounds, wherein two carbon-centered radicals are generated by the release of nitrogen gas, both reactions relying on thermal decomposition by homolytic bond cleavage of weak chemical bonds. Examples of classically used thermal initiators are shown in Scheme 1, along with their 10 hours half-life temperature. Because these compounds undergo thermal decomposition to generate the reactive radical species, radical processes are usually carried out at elevated temperature, *i.e.* above 70°C.

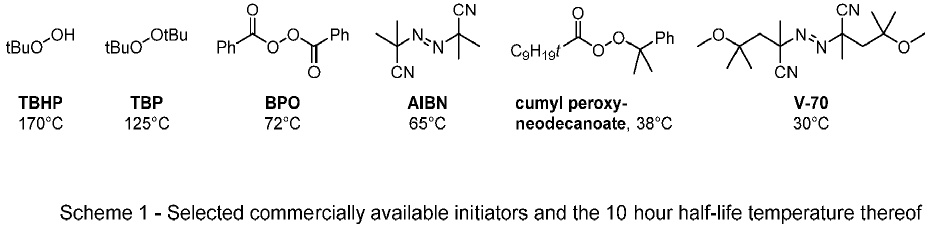

A large number of initiators have been developed over the years and are known to those skilled in the art. It is possible to conduct radical processes at a variety of temperatures by choosing a suitable initiator (see Scheme 1 for selected initiators with largely different 10 hours half-life temperatures). However, initiating radical processes at room temperature and below still remains a challenge. It is the purpose of the present invention to provide a safe, general and convenient solution to this problem.

If it is intended to conduct radical processes at room temperature or below, one has to use an initiator that decomposes at such temperatures with a sufficient rate. The desired initiator therefore has to be sufficiently unstable to decompose at relatively low temperature but needs to be stable enough to be produced, transported and handled without requiring extreme precautions. Two examples of such commercially available low temperature initiators are cumyl peroxyneodecanoate and the azobis compound V-70 which have 10 hour half-life temperatures of only 38°C and 30°C, respectively. While these are commercial and useful products, it is evident that such compounds are extremely hazardous, as illustrated by their respective Self Accelerating Decomposition Temperature (SADT) of 10°C and 30°C. Particular care therefore has to be taken during their production, handling and storage.

To alleviate the inherent safety hazards associated with the use of such unstable compounds, other strategies have been developed in the state of art to generate initiating free radical species at low temperature by external activation of relatively thermally stable compounds. As an example of such strategy, the sensitivity of peroxides and azobis compounds to UV light irradiation has been exploited for initiation purposes in the state of art. However, the quality of commercial solutions can vary and rapidly degrade if not stored under the right conditions. Therefore, finding reliable and robust radical initiation methods for low temperature radical reactions is still highly desirable and it is the purpose of the present invention to provide a safe, general and convenient solution to this problem.

Peroxyketals are well-known initiators in the context of polymer chemistry, for example in the curing of unsaturated polyester resins, and a variety of them are commercially available.

U.S. Patent No. 4,032,596 describes the combination of a perketal and quaternary ammonium salts for the acceleration of the radical curing of polyesters. No acids were used in this invention and the ammonium salt addition was for reducing the curing time at the reaction temperature of around 100°C.

Sheppard and Kamath (Polym. Eng. Sci. 1979, 19, 597) are mentioning the combination of a perketal and dichloroacetic acid for the radical curing of resins. The acid addition was intended for reducing the curing time at the reaction temperature of about 132°C.

U.S. Patent No. 4,376,841 describes the combination of geminal bisperoxides (perketals) and an acid or acid-releasing compound for the copolymerization of unsaturated polyester resins to generate undefined crosslinked polymers.

In Schweitzer et al. (Angew. Chem. Int. Ed. 2013, 52, 13228), the combination of a perketal and an acid for the oxidative dimerization of xanthene and the oxidative cross-coupling of xanthene with cyclopentanone is described, but the reaction is limited to forming these two products in medium yields.

The inventors now found out that free radicals can be easily and reliably generated by the combination of a certain type of organic peroxides and an acid catalyst. These radicals can then initiate radical processes at temperatures well below those needed to induce free radical formation thermally (by homolytic O-O bond cleavage) from these organic peroxides, for example at 0°C.

In the context of the invention, a free radical is defined, following the IUPAC definition, as a chemical entity having an unpaired electron and is not to be confused with the term radical often used to describe substituents on complex molecules. Similarly, a radical process or reaction is defined as a process or reaction involving or making use of free radicals as reaction intermediates or initiating species. In the rest of this document, an initiator system, or initiator, is to be understood as a chemical compound or combination of chemical compounds capable of generating initiating free radical species under relevant experimental conditions.

Thus, the present invention is directed to a process for carrying out a chemical radical reaction in which an initiator system capable of generating a radical species for initiating a variety of radical reactions at low temperature is used.

In more detail, the present invention refers to a process for carrying out a chemical reaction in which a compound capable of forming a chemical radical is reacted with a reaction partner in a radical reaction in the presence of said initiator system comprising at least an acid, preferably selected from a Broensted acid or a Lewis acid, and a compound of the general formula (I) as detailed below.

Said chemical reactions include, but are not limited to:
- Radical halogenations including the Wohl-Ziegler reaction; including reactions with bromine and chlorine, including reactions with other halide sources like hydrobromic acid N-bromosuccinimide, N-chlorosuccinimide, N-bromophthalimide, N-chlorophthalimide, or N-fluorobenzenesulfonamide; including reactions with substituted hydrocarbon substrates for the formation of benzylic, allylic or aliphatic halides.
- Radical reductions of halides and sulfides; including reactions with aryl halides, alkenyl halides, allylic halides, aliphatic halides, aliphatic aryl sulfides, and with hydrogen sources including tin, germanium and silicon hydride reagents, including tributyl tin hydride, tris(trimethylsilyl)silane, trichlorosilane, poly(methylhydride-siloxane) (PMHS), tertiary thiol or chloroform.
- Radical deoxygenation reactions; including the Barton-McCombie-deoxygenation, including the reaction of a xanthate or xanthic acid ester, a thiocarbonylimidazolyl ester or related dithiocarbonyl compounds with a hydrogen source including tin, germanium and silicon hydride reagents, including tributyl tin hydride, tris(trimethylsilyl)silane, trichlorosilane, diethylsilane, triethylsilane, ethyldimethylsilane, poly(methylhydride-siloxane) (PMHS), tertiary thiol or chloroform, to form the hydrocarbon.
- Radical decarboxylation reactions; including the Barton-decarboxylation; including the reaction of a carboxylic acid derivative, including a thiohydroxamate ester with a hydrogen source including tin, germanium and silicon hydride reagents, including tributyl tin hydride, tris(trimethylsilyl)silane, trichlorosilane, poly(methylhydride-siloxane) (PMHS), tertiary thiol or chloroform, to form the hydrocarbon.
- Radical polymerization reactions including co-polymerization of unsaturated monomers including styrenes, acrylates, methacrylates, acrylonitriles, acrylamides, vinyl acetates; vinyl halides, ethylenes, butadienes, including free-radical polymerization as well as controlled polymerizations following degenerative chain transfer mechanisms, e.g. RAFT or requiring a radical initiator as initiation source, such as for example reverse atom transfer radical polymerization R-ATRP)
- Thiol-ene reactions; including the addition of an alkyl thiol, aryl thiol, thiocarboxylic acid or dithiocarboxylic acid to a C-C multiple bond, including double bond and triple bond, forming sulfides, including aryl-alkyl and dialkyl sulfides, including products additionally containing one or more oxygen atoms like sulfoxides, sulfones or beta-hydroxy, beta-hydroperoxy and beta-keto-sulfides, and including polymers generated by addition of the above mentioned sulfur compounds to suitable unsaturated monomers.
- Radical Addition reactions; including atom transfer radical additions (ATRA), including the reaction of alkyl and aryl halides with compounds containing one or more C-C multiple bonds, including double bonds, aromatic systems (arenes and heteroarenes) and triple bonds, and C-heteroatom multiple bonds, including carbonyl compounds, imines, nitrones, isonitriles, azides and nitriles, resulting in the formal addition of the alkyl and aryl groups, respectively and the halide to the multiple bond, including intramolecular reactions resulting in cyclization.
- Reductive cyclization reactions, including the intramolecular reaction of alkyl and aryl halides with residues containing one or more C-C multiple bonds, including double bonds and triple bonds, and a hydrogen source including tin, germanium and silicon hydride reagents, including tributyl tin hydride, tris(trimethylsilyl)silane, trichlorosilane, poly(methylhydride-siloxane) (PMHS), tertiary thiol or chloroform, resulting in cyclization by the formal addition of the alkyl and aryl groups, respectively, and a hydrogen atom to the multiple bond.

The initiator system comprises at least two components: a peroxide and an acid catalyst.

The peroxide component has the general Formula (I): 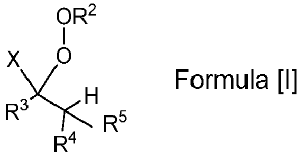 wherein:
X is selected from -OR¹, -OC(O)R¹, -OC(O)OR¹, -OOR¹, -NRR¹, -SR¹, -SSR¹,-OP(O)(OR)(OR¹), -OP(OR)(OR¹), -N₃, -NCO, -NCS, -CN, -N₃ or halogen; R, R¹ and R² each independently represent H, alkyl, cycloalkyl, heterocycloalkyl, alkenyl, alkynyl, aryl, heteroaryl, aralkyl, heteroaralkyl, acyl, sulfonyl, sulfinyl, phosphonate, phosphinate, silyl, silyloxy, each being optionally substituted by one or more groups selected from alkyl, cycloalkyl, heterocycloalkyl, alkenyl, alkynyl, heteroaryl, aralkyl, heteroaralkyl or heterosubstituent; or
R¹ and R² form a cyclic 3 to 20 membered ring structure which may further be substituted by alkyl, cycloalkyl, heterocycloalkyl, alkenyl, alkynyl, aryl, heteroaryl, or heterosubstituent groups; each being optionally substituted by one or more groups selected from alkyl, cycloalkyl, heterocycloalkyl, alkenyl, alkynyl, heteroaryl, aralkyl, heteroaralkyl or heterosubstituent or include heteroatoms, including peroxygroup(s), within the cyclic structure;
R³ is H, alkyl, cycloalkyl, heterocycloalkyl, aryl, alkenyl, alkynyl, heteroaryl, aralkyl, heteroaralkyl, acyl, sulfonyl, sulfinyl, phosphonyl, phosphinyl, silyl, silyloxyl, each being optionally substituted by one or more groups selected from alkyl, cycloalkyl, heterocycloalkyl, alkenyl, alkynyl, heteroaryl, aralkyl, heteroaralkyl or heterosubstituent;
R³, R⁴ and R⁵ each independently represent H, alkyl, cycloalkyl, heterocycloalkyl, alkenyl, alkynyl, aryl, heteroaryl, or heterosubstituent groups; each being optionally substituted by one or more alkyl, cycloalkyl, heterocycloalkyl, alkenyl, alkynyl, heteroaryl, aralkyl, heteroaralkyl or a heterosubstituent, or any two of R³, R⁴ and R⁵ form a cyclic C₂ to C₂₀ hydrocarbon structure which may further be substituted by alkyl, cycloalkyl, heterocycloalkyl, alkenyl, alkynyl, aryl, heteroaryl, or heterosubstituent groups; each being optionally substituted by one or more groups selected from alkyl, cycloalkyl, heterocycloalkyl, alkenyl, alkynyl, heteroaryl, aralkyl, heteroaralkyl or heterosubstituent or include heteroatoms, including peroxygroup(s), within the cyclic structure, and the remaining of R³, R⁴ and R⁵ have the meaning as given before.

The processes as disclosed in US 4,376,841 making use of geminal bisperoxides for the copolymerization of unsaturated polyester resins to generate crosslinked polymers and the processes of Schweitzer *(*Angew. Chem. Int. Ed. 2013, 52, 13228) are not part of the invention.

A heterosubstituent according to the invention is to be understood as a substituent including heteroatoms, preferentially selected from O, N, S, Si and halogens. It can be preferentially selected from, =O, -OH, -F, -Cl, -Br, -I, -CN, -N₃, -NO₂,-SO₃H, NCO, NCS, OP(O)(OR^{S1})(OR ^{S2}), OP(OR^{S1})(OR^{S2}), a monohalogenomethyl group, a dihalogenomethyl group, a trihalogenomethyl group, -CF(CF₃)₂, -SF₅,-NR^{S1}R^{S2}, -OR^{S1}, -OOR^{S1}, -OSiR^{S1}R^{S3}, -OSi(OR^{S1})R^{S2}R^{S3},--OSi(OR^{S1})(OR^{S2})R^{S3}, -OSi(OR^{S1})(OR^{S2})R^{S3}, -OSO₂R^{S1}, -SR^{S1}, -SSR^{S1},-S(O)R^{S1}, -S(O)₂R^{S1}, -C(O)OR^{S1}, -C(O)NR^{S1}R^{S2}, -NR^{S1}C(O)R^{S2}, -C(O)-R^{S1},-COOM, wherein M represents a metal such as Na, K or Cs.

R^{S1} R^{S2} and R^{S3} each individually represent H, alkyl, cycloalkyl, heterocycloalkyl, alkenyl, alkynyl, aryl, heteroaryl, aralkyl, heteroaralkyl, sulfonyl, silyl, each being optionally substituted by one or more alkyl, cycloalkyl, heterocycloalkyl, alkenyl, alkynyl, heteroaryl, aralkyl, heteroaralkyl, sulfonyl or heterosubstituent.

In a further embodiment of the invention, any two of R, R¹, R², R³, R⁴, R⁵, R^{S1} R^{S2} and R^{S3} together form a ring structure comprising 3 to 20 ring atoms which may further be substituted by alkyl, cycloalkyl, heterocycloalkyl, alkenyl, alkynyl, aryl, heteroaryl, or heterosubstituent groups; each being optionally substituted by one or more groups selected from alkyl, cycloalkyl, heterocycloalkyl, alkenyl, alkynyl, heteroaryl, aralkyl, heteroaralkyl or heterosubstituent or include heteroatoms, including peroxygroup(s), within the cyclic structure.

Any one of R¹, R², R³, R⁴, R⁵, R^{S1} R^{S2} and R^{S3} may be bound to a soluble polymer or a solid phase material, such as a polymeric or inorganic support.

In a preferred embodiment, the peroxide component of the general formula (I) comprises at least one peroxyketal group as represented in the general formula (I) wherein X is OOR¹ and R¹ to R⁵ have the same meaning as given before.

For the initiator system in more detail, alkyl may be C₁-C₂₀-Alkyl which can be straight chain or branched or cyclic and has 1, 2, 3, 4, 5, 6, 7, 8, 9, 10, 11, 12, 13, 14, 15, 16, 17, 18, 19 or 20 carbon atoms. Alkyl might particularly be C₁-C₆-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2-, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl. Substituted alkyl groups can be for example trifluoromethyl, pentafluoroethyl and 1,1,1-trifluoroethyl.

Cycloalkyl may be a cyclic alkyl group forming a 3 to 10 membered ring and might be cyclopropyl, cyclobutyl, cyclopentyl, cyclohexyl, cycloheptyl or cyclooctyl.

Heterocycloalkyl may be a cycloalkyl forming a 3 to 10 membered ring and incorporating one or more heteroatoms selected from N, O, S and Si within the cycle. In particular, heterocycloalkyls can be preferentially selected from 2,3-dihydro-2-, -3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetrahydro-1-, -2- or -4-imidazolyl, 2,3-dihydro-1-, -2-, -3-, -4- or -5-pyrazolyl, tetrahydro-1-, -3- or -4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4- or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7- or -8-quinolyl, 1,2,3,4-tetrahydro-1-, -2-, -3-, -4-, -5-, -6-, -7- or -8-isoquinolyl, 2-, 3-, 5-, 6-, 7- or 8-3,4-dihydro-2H-benzo-1,4-oxazinyl.

Alkenyl might be C₂-C₂₀ alkenyl.

Alkynyl might be C₂-C₂₀ alkynyl.

Halogen is F, Cl**,** Br or I.

Aryl might be phenyl, naphthyl or biphenyl and substituted derivatives thereof.

Aralkyl might be benzyl, naphthylmethyl and substituted derivatives thereof.

Heteroaryl may have one or more heteroatoms selected from N, O ,S and Si and is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4-or 5-thiazolyl, 3-, 4- or 5-isothiazolyl, 2-, 3- or 4-pyridyl, 2-, 4-, 5- or 6-pyrimidinyl, also preferably 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

Heteroaralkyl might be any of the aforementioned heteroaryl bound to an alkyl group, such as pyridinylmethyl.

Optionally substituted means unsubstituted or monosubstituted, disubstituted, trisubstituted, tetrasubstituted, pentasubstituted, or even further substituted on the respective group.

In a preferred embodiment, the peroxide component of the general formula (I) might be preferably selected from the group of compounds shown in Scheme 2.

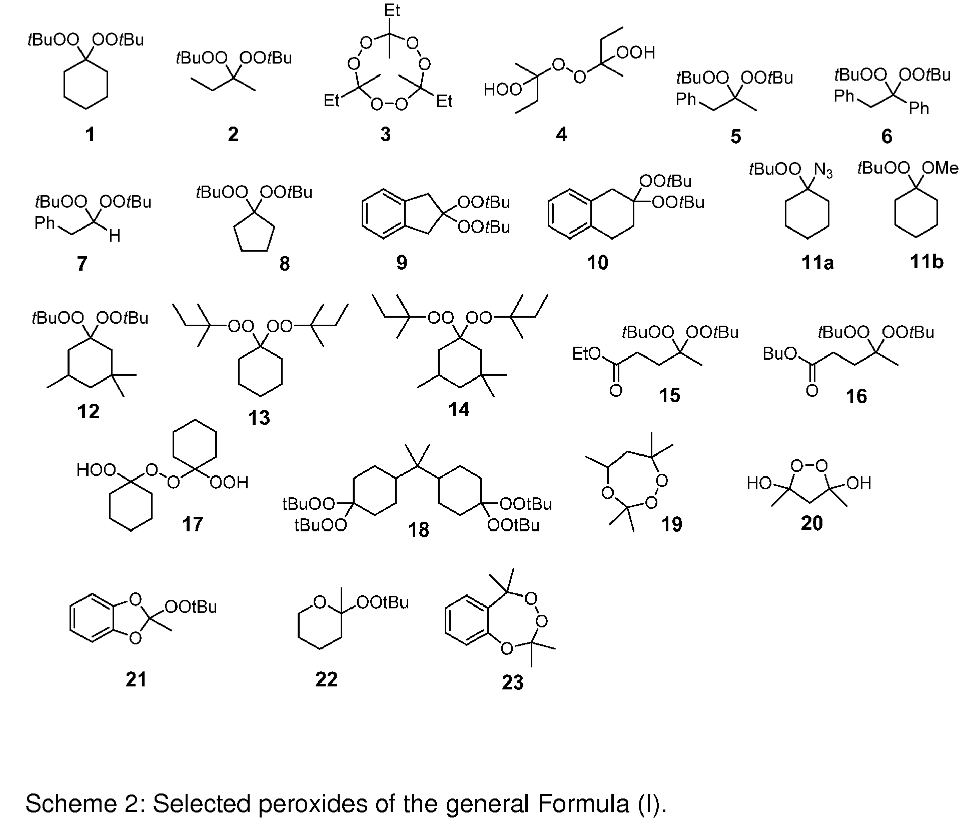

Further preferred are compounds 3, 4, 9, 10, 11 a, 11 b, 17, 18, 19, 20, 21 22 and 23.

The peroxide component of the initiator system may be introduced to the reacting system in pure form, as a solution in a solvent, formed *in-situ* from suitable precursors before the introduction of the acid catalyst component, as an non-purified mixture, for example of components required for its formation under commercially relevant conditions, or be present in residual amounts from a previous process, for example a radical process relying on its thermal decomposition, or as a mixture of several peroxides,

The acid catalyst component can be any compound with sufficient acidic properties. In particular, the acid catalyst can be an organic or mineral acid, often described as Brønsted acids, or a metal salt with Lewis acidic properties. The acid catalyst component can be used under homogeneous or heterogeneous conditions.

By homogeneous conditions, it is to be understood that the acid catalyst component is completely or partially soluble in the reaction medium.

By heterogeneous conditions, it is to be understood that the acid catalyst remains in a separate phase, typically a liquid or solid phase or immobilized on a support, from the reaction medium, allowing for example the recovery of the acid catalyst component at the end of the reaction, while the activation reaction occurs at the interface of these two separate phases.

In a preferred embodiment, the acid catalyst can be an organic or mineral acid having a pKa value in water of 4,75 or lower. Such acid might be for example trifluoroacetic acid, nitric acid, hydrochloric acid, sulfuric acid, methanesulfonic acid, para-toluenesulfonic acid, phosphoric acid, trifluoromethanesulfonic acid (triflic acid), 1,1,1-trifluoro-N-((trifluoromethyl)sulfonyl)methanesulfonamide (triflimide).

In another preferred embodiment, the acid catalyst can be a salt of an element selected from groups 1, 2, 3, 4, 12, 13, 14 and 15 of the periodic table. Such acid catalyst might be for example Scandium(III) triflate, Ytterbium(III) triflate, Titanium(IV) chloride, Hafnium(III) triflate, Zinc(II) chloride, Aluminium(III) chloride, Indium(III) triflate, Tin(II) chloride, Tin(IV) chloride, Bismuth(III) triflate.

In the inventive process, mixtures of peroxides having different reactivities and/or mixtures of acids having different pKa value can be used in order to control the free radical reaction.

The ratio of peroxide to acid catalyst components can vary widely from 1:100 to 100:1 depending on the particular radical process in which it is to be used.

Preferably, the ratio varies between 1:10 to 10:1. Typically, a 1:1 ratio is advantageously convenient.

The amount of initiator can again widely vary depending on the particular radical process being initiated. Preferred amounts can vary from catalytic amounts, for example from 0.01 mol% to 20 mol% to overstoichiometric amounts, as high as 1000 mol%.

The inventive initiator system is compatible with a wide variety of inert or reactive solvents. Therefore, solvents used in the inventive process are not particularly limited or even required and may be preferably selected from aliphatic, cycloaliphatic or aromatic solvents, chlorinated solvents, alcohols, in particular lower aliphatic alcohols, esters, ethers or mixtures thereof such as, for example, hexane, benzene, toluene, dichloromethane, chloroform, methanol, dimethylsulfoxide, acetic acid, acetonitrile, ethyl acetate or diethyl ether. The initiator system can also be used without any additional solvent, for example in the case of a radical polymerisation.

The reaction temperature is in general a temperature lower than the 10 hour half-life temperature of the peroxide component of general Formula (I) used. It can therefore be generally selected from any temperature below 100°C. In a preferred embodiment, the temperature is selected between -40°C and 60°C. Typically, reactions are advantageously run between 0°C and 25°C.

As assumed by the inventors and shown in Scheme 3, the radicals are formed from the peroxide component **(24)** in the presence of an acid catalyst component by the formation of an alkenyl peroxide **(25),** which is a generally very unstable structure that rapidly decomposes into radical **(26)** and an oxyl radical by O-O bond homolysis at low temperatures.

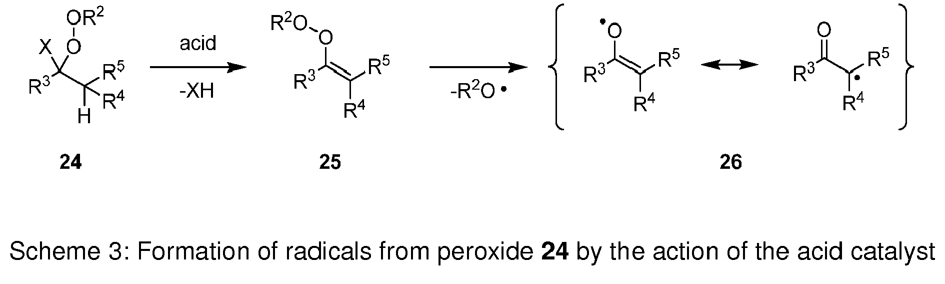

The so-generated free radicals can then be used as initiating species for many types of radical process known to those skilled in the art, including, for example, but not limited to, all as further exemplified above:
- Radical halogenations including the Wohl-Ziegler reaction;
- Radical reductions of halides ;
- Radical deoxygenation reactions;
- Reductive cyclization reactions;
- Radical polymerization reactions including co-polymerization of unsaturated monomers including styrenes, acrylates, methacrylates, acrylonitriles, acrylamides, vinyl acetates; vinyl halides, ethylenes, butadienes, including free-radical polymerization as well as controlled polymerizations following degenerative chain transfer mechanisms, e.g. RAFT or requiring a radical initiator as initiation source, such as for example reverse atom transfer radical polymerization R-ATRP)
- Thiol-ene reactions.
- Radical Addition reactions.

The examples detailed by the inventors below only serve to demonstrate the generality and utility of the initiator system for the processes described in the present invention. It is however to be understood that these examples serve only for illustration purposes and do not limit the breadth of applications covered by the present invention. Those skilled in the art will easily recognize the potential of the present invention to be used for the initiation of a variety of other radical processes at low temperature not described by the inventors in the following examples. Such radical processes are for example radical deoxygenations, known as the Barton-McCombie reaction; polymerisation of a wide variety of olefinic monomers, such as vinyl acetates, acrylonitriles, butadiene and the like; or the wide variety of radical chain reactions relying on metal-hydride, such as tin, germanium or silicon hydrides, or sulphur based radical transfer agents.

According to the invention, the peroxides which can be used according to the invention are not particularly limited as long as they are covered by the formula (I) as represented above. Compounds that have been exemplarily used by the inventors are peroxides **1-11b,** shown in Scheme 2. Some of these compounds as well as derivatives thereof are commercially available.

Thus, the inventive initiator system can be particularly used as a complementary strategy to the existing radical initiators. Said initiator system can be used to efficiently initiate radical processes at ambient temperature, temperatures as low as -20°C or even lower. This combination has the potential to become an extremely useful and widely used system, given the commercial availability of several peroxides, their tolerance to air and moisture, their high thermal stability, compared to specifically designed low temperature initiators, and the extreme simplicity of the experimental procedure.

The introduction of an activation catalyst as found by the inventors offers the opportunity to finely tune the rate of formation of free radicals from a specific peroxide based on the nature or the concentration of the acid component. It therefore presents an attractive alternative to the use of syringe pump techniques that are often used in the context of organic synthesis to keep the concentration of initiating free radicals low.

The invention is further illustrated by the following Examples.

### Example 1: Radical bromination of benzylic compounds

The inventors selected the radical bromination of fluorene **27** by *N-*bromosuccinimide (NBS) at room temperature as a benchmark reaction to evaluate their inventive initiator system (Scheme 4).

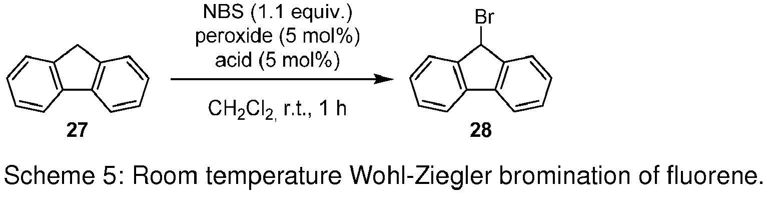

All reactions were performed according to the following procedure: In an oven-dried Schlenk flask fluorene **27** (83 mg, 0.5 mmol, 1 eq) and *N*-bromosuccinimide (98 mg, 0.55 mmol, 1.1 eq) were dissolved in dichloromethane (5 mL). The desired peroxide (0.025 mmol, 5 mol %) was introduced and the resulting mixture was degassed by the freeze-pump-thaw method (3 cycles). After warming to room temperature, the acid catalyst was added under a stream of argon and after the desired reaction time, the reaction mixture was quenched with NEt₃ (250 µL), CH₂Br₂ (0.5 mmol) was added as a standard for analytical purposes only and an aliquot taken for direct ¹H NMR analysis. Yield was determined by integrating a reference peak of **28** (5.9 ppm, s, 1 H; determined from an authentic sample) relative to the peak of CH₂Br₂. The results of the reactions are detailed in (Table 1).

**Table 1: Wohl-Ziegler bromination of fluorene at room temperature.**

| Entry | Peroxide | Acid catalyst | Yield of **28** (%) |
|---|---|---|---|
| 1 | **1** | H₂SO₄ | 72 |
| 2 | **1** | pTsOH | 67 |
| 3 | **1** | CH₃SO₃H | 45 |
| 4 | **1** | HNO₃ | 96 |
| 5 | **1** | CF₃CO₂H | 22 |
| 6 | **1** | CCl₃CO₂H | 18 |
| 7 | **1** | AcOH | 0 |
| 8 | **1** | Sc(OTf)₃ | 69^{[a]} |
| 9 | **11b** | CH₃SO₃H | 21 |
| 10 | **11a** | CH₃SO₃H | 47 |
| 11 | **2** | CH₃SO₃H | 10; 76^{[b]} |
| 12 | **5** | CH₃SO₃H | 20 |
| 13 | **6** | CH₃SO₃H | 0; 33^{[b]} |
| 14 | **7** | CH₃SO₃H | 0; 9^{[b]} |
| 15 | **3** | CH₃SO₃H | 0; 8^{[b]} |
| 16 | **4** | CH₃SO₃H | 1; 12^{[b]} |
| 17 | **9** | CH₃SO₃H | 50 |
| 18 | **10** | CH₃SO₃H | 74 |

| | | | |
|---|---|---|---|
| [a] reaction performed in acetonitrile as solvent; [b]After 72 hours of reaction | | | |

The bromination proceeded efficiently using a commercial solution of peroxyketal **1** (Trigonox® 22, 50% weight in mineral oil) in combination with different Brønsted acids. Control experiments confirmed the requirement for both acid and peroxide, no conversion being observed after 24 hours if either one of these components was omitted. A clear trend following the p*K*ₐ value of the acid catalyst can be seen: stronger acids give faster conversion.

Sulfuric and para-toluene sulfonic acid have similar behaviour with 72% and 67% of **28** after one hour, respectively (entries 1 and 2). Methane sulfonic acid gave a slightly lower yield (45%; entry 3) while acids weaker than trifluoroacetic acid (22%, entry 5) or trichloroacetic acid (18%, entry 6) failed to give any conversion (entry 7). Nitric acid is more efficient than its p*K*ₐ value would suggest (96%; entry 4). Eventually, all reactions gave high yields when allowed to reach full conversion (80-95% yield of **28** after 24 to 72 hours), showing that the acid catalyst only influences the initiation rate. Scandium (III) triflate, a Lewis acid, was also found to be competent (69%; entry 8).

Different commercial peroxyketal solutions were evaluated using methane sulfonic acid as a standard catalyst of medium reactivity. **2** (Trigonox® D; 50% weight) proved to be less efficient than **1** (45%, entry 3), giving 10% of product **28** after one hour and 76% after 48 hours (entry 11). **3** (Trigonox® 301; 41% weight) showed low conversion after two days of reaction (8%, entry 15). **4** (Luperox® DHD-9, 32% weight) was found to be slightly more reactive than **3,** giving 12% product after 48 hours (entry 16).

Based on the observation of this strong influence of the peroxyketal structure on its reactivity, the inventors evaluated a series of structurally different peroxides. The effect of the group X of Formula (I) is shown by compounds **11a** and **11b. 11b** proved to be less reactive than **1** (21%, entry 9) while **11a** was more efficient, giving 47% of **28** after one hour (entry 10). Aromatic substituents around the peroxide moiety can have significant effects: **5** is more effective than **2** (20%, entry 12 compared to entry 11), while **6** was much less efficient (33% after 48 h, entry 13) **9** was found to be slightly more reactive than **1** (50%, entry 17) while **10** was the most efficient of the structures evaluated, giving 74% of **28** after one hour of reaction (entry 18).

The inventive initiator system was then evaluated at cryogenic temperatures using methane sulfonic acid as a standard acid and varying the peroxide component of the initiator system.

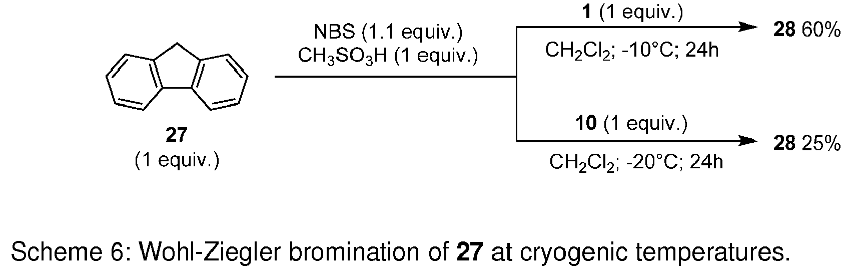

Performing the reaction at -10°C, using a stoichiometric amount of **1** and methane sulfonic acid, a 60% yield of **28** was obtained after 24 hours., With peroxide **10,** the reaction could be performed successfully at -20°C, giving 25% of product **28** after 24 hours (Scheme 6). These results show that the efficiency of the initiation system can be improved by the right selection and combination of peroxide component and acid catalyst component and tuned for specific applications wherein fast or slow initiation rates or the use of cryogenic temperatures are required.

### Example 2: Bulk polymerization of methyl methacrylate:

Bulk polymerization of methyl methacrylate (MMA) was performed using the inventive initiator system at room temperature by the following procedure.

All tests were performed under air by dissolving the corresponding peroxide solution (10 µL, 1 vol %) in 1 mL of MMA (containing 1 mg.mL-1 of 9-nitronanthracene as colorant for better visualization) and adding methane sulfonic acid (5 µL, 0.5 vol %). The vial was left to stand until a glass-like solid was obtained. Full polymerization was considered achieved when a solid glass-like solid was obtained with low residual monomer odor. The times required for full polymerization are summarized in Table 2.

**Table 2: Bulk Polymerisation of MMA at Room Temperature.**

| Entry | Peroxide | Additive | Time (h) |
|---|---|---|---|
| 1 | **1** | - | 6 |
| 2 | **2** | - | 22 |
| 3 | **3** | - | 70 |
| 4 | **4** | - | 46 |
| 5 | **1** | MeOH (10 µL) | 9 |
| 6 | **2** | MeOH (10 µL) | 50 |
| 7 | **3** | MeOH (10 µL) | 70 |
| 8 | **4** | MeOH (10 µL) | 22 |

All peroxide components evaluated successfully initiated the polymerization of MMA at room temperature in varying amounts of time. The observed order of reactivity of peroxide components is **1** (6 hours, entry 1) > **2** (22 hours, entry 2) > **4** (46 hours, entry 4) > **3** (70 hours, entry 3), as expected from the results of Example 1.

The presence of small amounts of methanol as additive slowed the polymerisation of MMA with peroxides **1** and **2** (6h, entry 1 Vs 9h, entry 5 for **1;** 22h (entry 2 Vs 50h, entry 6 for **2**) but accelerated it for peroxide **4** (46h, entry 3 Vs 22h, entry 8).

### Example 3: Controlled Polymerizations of unsaturated monomers

Examples for radical polymerization reactions that have been conducted making use of the inventive initiator system include the synthesis of poly(n-butyl acrylate), poly(n-butyl acrylate)-b-poly(t-butyl acrylate) (a block copolymer), poly(styrene) and poly(N-isopropylacrylamide). These reactions have been carried out at ambient temperature and 0°C, respectively. These polymers have been synthesized with and without the technique of reversible addition fragmentation transfer (RAFT) polymerization.

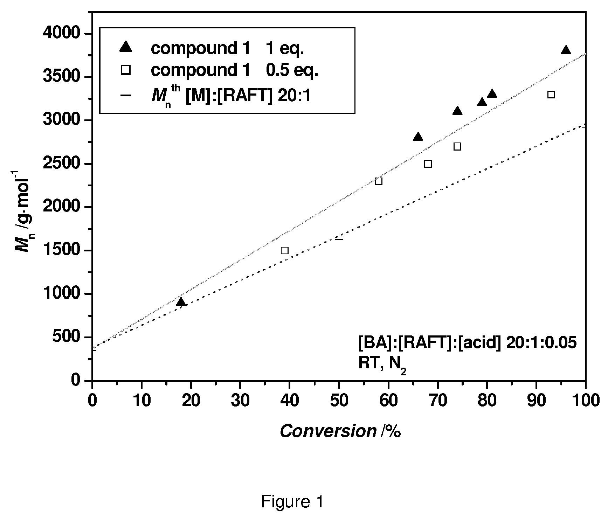

Peroxide **1** can be efficiently used to initiate radical polymerizations when being combined with a Brønsted acid at low temperature with any radically polymerizable vinyl monomer, such as for example styrene, butyl acrylate or N-isopropyl acrylamide. Polymers of high molecular weight are obtained in uncontrolled radical polymerizations in accordance with general expectations of such reactions. Initiation pathways have been identified and the initiating moieties do not correspond to a thermal decay of **1,** but to the fragments as outlined in Scheme 4. Furthermore, **1** combined with an acid (e.g. trifluoroacetic acid) can be used to initiate also room temperature degenerative transfer polymerizations, i.e. reversible addition fragmentation radical transfer polymerization (RAFT). Good control over a series of polymerizations is achievable, also allowing for block copolymer synthesis. Polymers show low dispersities (1.1-1.3) and the average degree of polymerization increases linearly with increasing monomer conversion. A good end-group functionality is detected via soft-ionization mass spectrometry. The evolution of number-average molecular weight of poly(n-butyl acrylate) with increasing monomer conversion obtained from room temperature RAFT polymerization employing **1** is illustrated in Figure 1.

A typical experimental procedure for polymerisation is as follows:
10 mmol (20 equiv) of monomer, 0.5 mmol (1 equiv) of DoPAT RAFT agent, when applicable, and 0.5 mmol (1 equiv) of **1** were added into a sealed glass vial containing a stirring bar which was purged with nitrogen for 10 min and subsequently inserted in a glovebox under inert atmosphere. The reaction was started by adding 0.025 mmol (0.05 equiv.) of para-toluene sulfonic acid, dissolved in 0.1 mL of acetonitrile. The mixture was polymerized at the desired temperature inside the glove-box. Samples were taken and quenched with hydroquinone/methanol after specific reaction times to determine conversion (by NMR) and molar mass (by SEC). After 24 hours, the residual reaction mixture was quenched by adding a solution of hydroquinone (1 mmol, 2 equiv) in methanol and poured into an aluminium pan to evaporate any residual monomer and solvent. Molar mass distribution and end group fidelity were determined by THF-SEC and ESI-MS analysis.

### Example 4: Thiol-ene reaction

Addition of a thiol to a terminal olefin proceeded smoothly in the presence of 20 mol% of **1** and 10 mol% methanesulfonic acid to give the thiol-ene addition product **29** in 88% yield (Scheme 7).

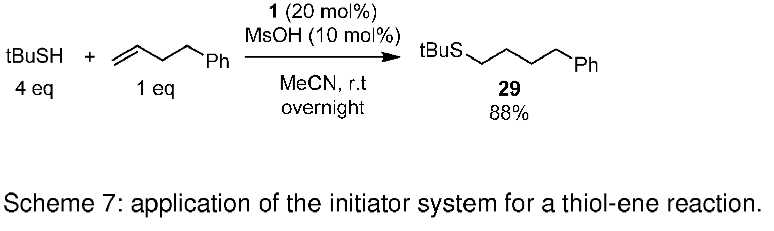

In an oven dried Schlenk tube, tBuSH (225µL, 2 mmol), 4-phenyl butane (75µL, 0.5 mmol) and **1** (50% solution, 52 mg, 0.1 mmol) were dissolved in acetonitrile (5 mL). The resulting mixture was degassed (Freeze-Pump-Thaw technique, 3 cycles), brought to room temperature and methane sulfonic acid (3.5 µL, 0.05 mmol) was added and the mixture left to react overnight. The mixture was transferred to an extraction funnel, diluted with ethyl acetate (20 mL) and washed with NaOH (2M, 2x 10 mL) and distilled water (2x 10 mL). The organic phase was dried over Na₂SO₄, evaporated to dryness and the resulting oil was purified by flash chromatography on silica gel (Hex/AcOEt 99:1 as eluent) to afford **29** as a clear oil (98mg, 88% yield).

### Example 5: Radical Addition reaction

Radical addition (ATRA) of carbon tetrachloride was successfully initiated. Although it required a larger amount of **1,** 61% of product **30** was obtained (Scheme 8).

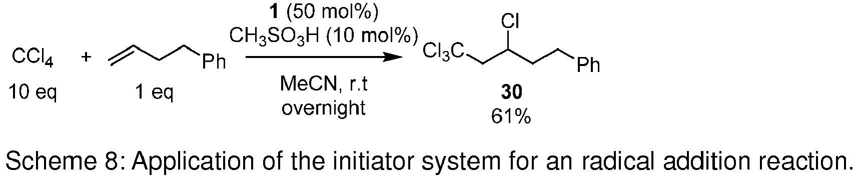

In an oven dried Schlenk tube, CCl₄ (962µL, 10 mmol), 4-phenyl butane (150µL, 1 mmol) and **1** (50% solution, 260 mg, 0.5 mmol) were dissolved in acetonitrile (1 mL). The resulting mixture was degassed (Freeze-Pump-Thaw technique, 3 cycles), brought to room temperature and methane sulfonic acid (7 µL, 0.1 mmol) was added and the mixture left to react overnight. The mixture was evaporated to dryness and the resulting oil was purified by flash chromatography on silica gel (hexane as eluent) to afford **30** as a clear oil (175 mg, 61% yield).

### Example 6: Radical dehalogenation reaction

The TMS₃SiH mediated reduction of 9-iodophenanthrene was performed, giving phenanthrene **31** in an excellent 94% yield (Scheme 9).

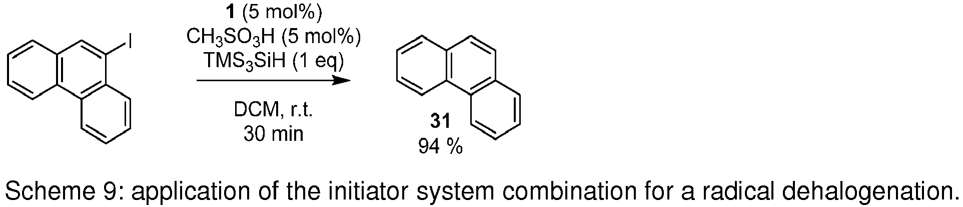

In an oven dried Schlenk tube, 9-iodophenanthrene (304 mg, 1 mmol), was dissolved in dichloromethane (10 mL). The resulting solution was degassed (Freeze-Pump-Thaw technique, 3 cycles), brought to room temperature and **1** (50% solution, 26 mg, 0.05 mmol) and TMS₃SiH (308 µL, 1 mmol) were added.

The mixture was degassed once more and after being brought back to room temperature, methane sulfonic acid (3.5 µL, 0.05 mmol) was added and the mixture left to react for 30 minutes. The mixture was evaporated to dryness and the resulting slightly yellow oil was purified by flash chromatography on silica gel (hexane as eluent) to afford **31** as a white solid (168 mg, 94% yield).

### Example 7: Reductive radical cyclisation

The reductive cyclization of **32** was successful, giving benzofurane **33** in 79% isolated yield (Scheme 10).

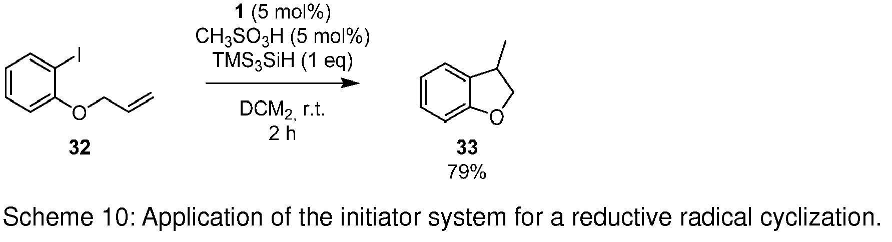

In an oven dried Schlenk tube, **32** (259 mg, 1 mmol), was dissolved in dichloromethane (10 mL). The resulting solution was degassed (Freeze-Pump-Thaw technique, 3 cycles), brought to room temperature and **1** (50% solution, 26 mg, 0.05 mmol) and TMS₃SiH (308 µL, 1 mmol) were added. The mixture was degassed once more and after being brought back to room temperature, methane sulfonic acid (3.5 µL, 0.05 mmol) was added and the mixture left to react for 2 hours. The mixture was evaporated to dryness and the resulting slightly yellow oil was purified by flash chromatography on silica gel (pentane as eluent) to afford **33** as a clear oil (106 mg, 79% yield)

These different examples demonstrate the generality of the inventive initiator system for low temperature radical processes, including cryogenic conditions. Abstraction of a hydrogen atom from suitable substrates is possible, as shown by the success of the Wohl-Ziegler and thiol-ene reactions. Direct abstraction of halogen atoms, presumably by the complementary carbon centred radical formed, is also possible, as in the ATRA of CCl₄. If the radicals generated are not reactive enough to initiate chains themselves, they are competent in initiating reactions relying on the use of a hydride mediator like TMS₃SiH. Besides these examples oriented towards organic synthesis, radical polymerization of several olefin monomers is successfully initiated at room temperature and below. The present invention could therefore be extremely valuable for applications where low temperatures are required.

The present invention offers a cheap, safe and user-friendly alternative to the low temperature radical initiators currently known.

## Claims

1. Process for carrying out a chemical reaction in which a compound capable of forming a chemical radical is reacted with a reaction partner at a temperature of below 100°C in a radical reaction in the presence of reaction pair of an acid, preferably selected from a Broensted acid or a Lewis acid, and of a compound of the general formula (I): 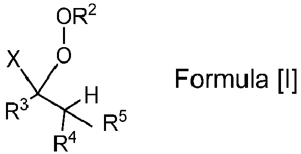 wherein:
X is selected from -OR¹, -OC(O)R¹, -OC(O)OR¹, -OOR¹, -NRR¹,-SR¹, -SSR¹, -OP(O)(OR)(OR¹), -OP(OR)(OR¹), -N₃, -NCO, -NCS, -CN, -N₃ or halogen;
R, R¹ and R² each independently represent H, alkyl, cycloalkyl, heterocycloalkyl, alkenyl, alkynyl, aryl, heteroaryl, aralkyl, heteroaralkyl, acyl, sulfonyl, sulfinyl, phosphonate, phosphinate, silyl, silyloxy, each being optionally substituted by one or more groups selected from alkyl, cycloalkyl, heterocycloalkyl, alkenyl, alkynyl, heteroaryl, aralkyl, heteroaralkyl or heterosubstituent; or
R¹ and R² form a cyclic 3 to 20 membered ring structure which may further be substituted by alkyl, cycloalkyl, heterocycloalkyl, alkenyl, alkynyl, aryl, heteroaryl, or heterosubstituent groups; each being optionally substituted by one or more groups selected from alkyl, cycloalkyl, heterocycloalkyl, alkenyl, alkynyl, heteroaryl, aralkyl, heteroaralkyl or heterosubstituent or include heteroatoms, including peroxygroup(s), within the cyclic structure;
R³ is H, alkyl, cycloalkyl, heterocycloalkyl, aryl, alkenyl, alkynyl, heteroaryl, aralkyl, heteroaralkyl, acyl, sulfonyl, sulfinyl, phosphonyl, phosphinyl, silyl, silyloxyl, each being optionally substituted by one or more groups selected from alkyl, cycloalkyl, heterocycloalkyl, alkenyl, alkynyl, heteroaryl, aralkyl, heteroaralkyl or heterosubstituent;
R³, R⁴ and R⁵ each independently represent H, alkyl, cycloalkyl, heterocycloalkyl, alkenyl, alkynyl, aryl, heteroaryl, or heterosubstituent groups; each being optionally substituted by one or more alkyl, cycloalkyl, heterocycloalkyl, alkenyl, alkynyl, heteroaryl, aralkyl, heteroaralkyl or a heterosubstituent, or any two of R³, R⁴ and R⁵ form a cyclic C₂ to C₂₀ hydrocarbon structure which may further be substituted by alkyl, cycloalkyl, heterocycloalkyl, alkenyl, alkynyl, aryl, heteroaryl, or heterosubstituent groups; each being optionally substituted by one or more groups selected from alkyl, cycloalkyl, heterocycloalkyl, alkenyl, alkynyl, heteroaryl, aralkyl, heteroaralkyl or heterosubstituent or include heteroatoms, including peroxygroup(s), within the cyclic structure, and the remaining of R³, R⁴ and
R⁵ have the meaning as given before.

2. Process according to claim 1, wherein the peroxide compound is represented by the general formula (I) wherein X is OOR¹ and R¹ to R⁵ have the meaning as defined in claim 1.

3. Process according to any of claims 1 to 2, wherein the peroxide compound of general formula (I) is selected from the group consisting of

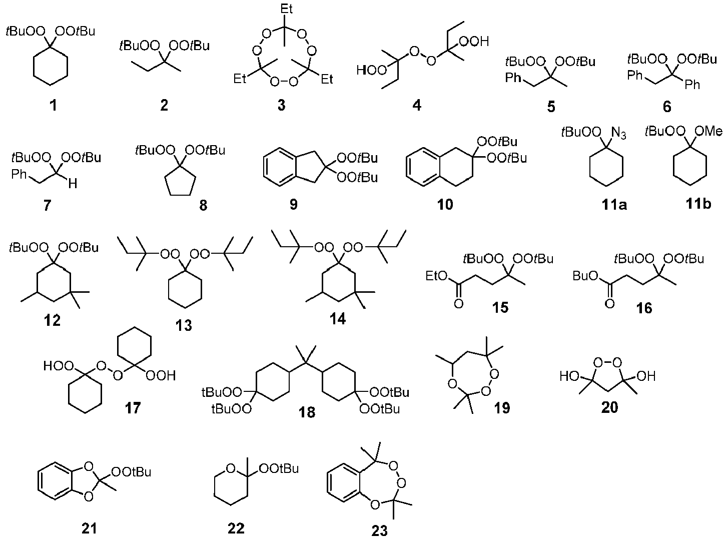

4. Process according to any of claims 1 to 3, wherein the acid component is an organic or mineral acid or a metal salt with Lewis acidic properties.

5. Process according to any of claims 1 to 4, wherein the acid catalyst is an organic or mineral acid having a pKa in water of 4,75 or lower.

6. Process according to any of claims 1 to 5 wherein the acid catalyst is selected from trifluoroacetic acid, nitric acid, hydrochloric acid, sulfuric acid, methanesulfonic acid, para-toluenesulfonic acid, phosphoric acid, trifluoromethanesulfonic acid (triflic acid), 1,1,1-trifluoro-N-((trifluoromethyl)sulfonyl)methanesulfonamide (triflimide).

7. Process according to any of claims 1 to 3, wherein the acid catalyst is a salt of an element selected from group 1, 2, 3, 4, 12, 13, 14 and 15 of the Periodic Table.

8. Process according to claim 7, wherein the acid catalyst is selected from Scandium(III) triflate, Ytterbium(III) triflate, Titanium(IV) chloride, Hafnium(III) triflate, Zinc(II) chloride, Aluminium(III) chloride, Indium(III) triflate, Tin(II) chloride, Tin(IV) chloride, Bismuth(III) triflate.

9. Process according to any of claims 1 to 8 wherein the peroxide component of general formula (I) and the acid component are used in a ratio from 1:100 to 100:1, preferably in a ratio between 1:5 to 5:1.

10. Process according to any of claims 1 to 9, wherein the peroxide component of general formula (I) is used in pure form, as a solution in a solvent, formed *in-situ* from suitable precursors before the introduction of the acid catalyst component, as an unpurified mixture of components required for its formation.

11. Process according to any of claims 1 to 10, wherein the acid catalyst component is used under homogeneous conditions such that it is completely or partially soluble in the reaction medium.

12. Process according to any of claims 1 to 10, wherein the acid catalyst component is used under heterogeneous conditions in a separate liquid or solid phase or immobilized on a support.

13. Process according to any of claims 1 to 12, wherein the radical reaction is selected from the group comprising of:
• Radical halogenations including the Wohl-Ziegler reaction;
• Radical reductions of halides ;
• Radical deoxygenation reactions;
• Reductive cyclization reactions;
• Radical polymerization reactions including co-polymerization of unsaturated monomers including styrenes, acrylates, methacrylates, acrylonitriles, acrylamides, vinyl acetates; vinyl halides, ethylenes, butadienes, including free-radical polymerization as well as controlled polymerizations following degenerative chain transfer mechanisms, including RAFT or requiring a radical initiator as initiation source, including reverse atom transfer radical polymerization R-ATRP);
• Thiol-ene reactions;
• Radical addition reactions;
each reaction carried out at temperatures below 100°C and preferably below 40°C.

14. Radical initiator system comprising at least one acid, preferably selected from a Broensted acid or a Lewis acid, and at least one peroxide compound of the general formula (I) 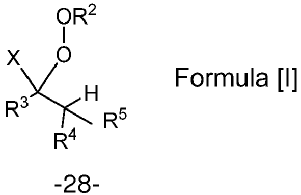 wherein X and R² to R⁵ have the meanings as given in any of the claims above.

15. Use of the initiator system of claim 14 in radical-initiated reactions wherein the radical reaction is selected from the group comprising of:
• Radical halogenations including the Wohl-Ziegler reaction;
• Radical reductions of halides ;
• Radical deoxygenation reactions;
• Reductive cyclization reactions;
• Radical polymerization reactions including co-polymerization of unsaturated monomers including styrenes, acrylates, methacrylates, acrylonitriles, acrylamides, vinyl acetates; vinyl halides, ethylenes, butadienes, including free-radical polymerization as well as controlled polymerizations following degenerative chain transfer mechanisms, e.g. RAFT or requiring a radical initiator as initiation source, such as for example reverse atom transfer radical polymerization R-ATRP)
• Thiol-ene reactions;
• Radical addition reactions.
